(19) Europäisches Patentamt
European Patent Office
Office européen des brevets

(11) Veröffentlichungsnummer: **0 248 359**
**A1**

(12) **EUROPÄISCHE PATENTANMELDUNG**

(21) Anmeldenummer: **87107795.4**

(22) Anmeldetag: **29.05.87**

(51) Int. Cl.4: **C12N 15/00** , C07K 15/14 , C12P 21/00 , A61K 37/02 , //(C12N15/00,C12R1:85),(C12N-15/00,C12R1:865)

(30) Priorität: **03.06.86 DE 3618638**

(43) Veröffentlichungstag der Anmeldung: **09.12.87 Patentblatt 87/50**

(84) Benannte Vertragsstaaten: **AT BE CH DE ES FR GB IT LI LU NL SE**

(71) Anmelder: **BEHRINGWERKE Aktiengesellschaft**
**Postfach 1140**
**D-3550 Marburg 1(DE)**

(72) Erfinder: **Zettlmeissl, Gerd, Dr.**
**Am Hofacker 15**
**D-3551 Lahntal(DE)**
Erfinder: **Bröker, Michael, Dr.**
**Lindenweg 16**
**D-3550 Marburg(DE)**
Erfinder: **Ragg, Hermann, Dr.**
**Am Kirchplatz 14**
**D-6233 Kelkheim (Taunus)(DE)**

(74) Vertreter: **Meyer-Dulheuer, Karl-Hermann, Dr. et al**
**HOECHST Aktiengesellschaft Zentrale Patentabteilung Postfach 80 03 20**
**D-6230 Frankfurt/Main 80(DE)**

(54) **Verfahren zur Sekretion von Proteinen aus Hefen.**

(57) Die cDNA für humanes Antithrombin III (AT III) bewirkt in Hefen eine Sekretion des Produktes. Die Signalsequenz für humanes AT III eignet sich somit zur Sekretion von Proteinen aus Hefen. Man erhält so ein biologisch aktives AT III, das immunologisch mit einem aus Plasma gewonnen AT III identisch ist. Das Produkt eignet sich daher zur Herstellung von Arzneimitteln.

EP 0 248 359 A1

Xerox Copy Centre

# Verfahren zur Sekretion von Proteinen aus Hefen

Die Erfindung betrifft die Sekretion von gentechnisch gewonnenen Proteinen aus Hefen als Wirtszellen, wobei die Sekretion dadurch bewirkt wird, daß als Signalsequenz diejenige für humanes Antithrombin III (AT III) dient.

Der Erfindung liegt die überraschende Erkenntnis zugrunde, daß AT III in Hefezellen sehr effektiv prozessiert wird und zum größten Teil in das Fermentationsmedium ausgeschieden wird.

Ein Aspekt der Erfindung betrifft ein Verfahren zur Sekretion von Proteinen aus Hefen, das dadurch gekennzeichnet ist, daß man in einer Hefe eine Genstruktur zur Expression bringt, in der vor dem Strukturgen für das gewünschte Protein die Signalsequenz für humanes AT III angeordnet ist. Vorteilhaft wird als Strukturgen die cDNA-Sequenz für AT III eingesetzt. Bevorzugt sind Hefen der Art Saccharomyces cerevisiae oder Schizosaccharomyces pombe.

Ein weiterer Aspekt der Erfindung ist biologisch aktives AT III, das nach dem erfindungsgemäßen Verfahren erhältlich ist. Dieses Produkt ist hefetypisch glykosyliert. Es ist immunologisch identisch mit AT III, das aus humanem Plasma isoliert wurde. Im Progressivinhibitortest zeigt das Produkt biologische Aktivität. Außerdem zeigt es Heparin Cofaktor-Aktivität.

Die Erfindung betrifft weiterhin Arzneimittel, die das erfindungsgemäß erhaltene AT III enthalten.

Weitere Aspekte der Erfindung und deren bevorzugte Ausgestaltungen werden im folgenden näher erläutert bzw. in den Patentansprüchen definiert.

Die für AT III codierende cDNA wurde aus einer Leber-cDNA-Genbank gewonnen.

Zur Expression in der Bäckerhefe Saccharomyces cerevisiae wurden Vektoren eingesetzt, die als Promotor einerseits den Alkoholdehydrogenase I-Promotor (ADH I) enthielten, wobei von dem bekannten Vektor pAAH5 (Ammerer, Methods in Enzymology 101 (1983) 192 - 201) Gebrauch gemacht wurde. Andererseits wurden Vektoren mit dem Cytochrom I-Promotor (cyc I) benutzt, wofür als Ausgangsplasmid pEX-2 (Ernst und Chan, J. Bacteriol. 163 (1985) 8 - 14) diente. Es zeigte sich, daß diese Promotoren auch in anderen Hefegattungen aktiv sind. Russell hat in Nature 301 (1983) 167 - 169 beschrieben, daß in vitro die Transkriptionsstartpunkte der S. cerevisiae ADH I-und cycI-Promotoren in S. pombe nicht erkannt werden und somit in S. pombe von den ADH I-und cycI-Promotoren mRNA gebildet wird, die nicht identisch mit der in S. cerevisiae synthetisierten mRNA ist. Trotz dieser in vitro-Befunde konnte gezeigt werden, daß die S. cerevisiae-ADH I-und cycI-Promotoren in S. pombe benutzt werden können, um eine heterologe Genexpression zu veranlassen (Tab. 1). In S. pombe $Ura^-$ (pMB218) und S. pombe $Ura^-$ (pMB 220) konnte mittels des AT III-spezifischen ELISA die Synthese von AT III nachgewiesen werden. Bei Fermentation im YPD-Medium sezernierten die Zellen in batch-Kultur bis zu 70 ng AT III/ml. Somit wird die humane AT III-Signalsequenz auch von S. pombe erkannt.

Da rekombinante Plasmide in Hefen nur unter Selektionsdruck stabil repliziert werden, kann erfindungsgemäß die AT III-cDNA auch in des Hefechromosom integriert werden. Diese Integration kann mit Hilfe des bekannten Plasmids YEp13 (Broach et al., Gene 8 (1979) 121 - 133) erfolgen. Hierzu wird die AT III-cDNA benachbart zum Leu2-Strukturgen eingebaut. Nach Transformation mit dem entsprechenden DNA-Fragment können Klone isoliert werden, die durch Rekombinationsereignisse den $Leu^+$-Phänotyp aufweisen. Alle Klone erwiesen sich als stabil hinsichtlich der Leu-Prototrophie und der AT III-Synthese und können ohne Verlust dieser Eigenschaften in nicht selektiven Medien fermentiert werden. Auch in diesem Falle wird das AT III zum größten Teil in das Medium sezerniert.

Zur Klärung, ob die Sezernierung tatsächlich darauf beruht, daß die Signalsequenz des humanen AT III von Hefen richtig erkannt wird oder ob womöglich die Struktur des Proteins hierfür verantwortlich ist, wurden Vektoren konstruiert, die ein AT III ohne eigene Signalsequenz codieren. Es zeigte sich, daß das AT III lediglich intrazellulär gebildet und nicht an das Medium abgegeben wird.

Zur Reinigung des Verfahrensproduktes wurde eine Anreicherung vorgenommen. Hierzu wurden die Überstände der Hefekulturen einer Affinitätschromatographie an Heparin-SEPHAROSE$^{(R)}$unterzogen (Miller-Anderssen et al., Thromb. Res. 5 (1974) 439 - 452). Das AT III wurde mit 1M NaCl eluiert. Somit entsprach das physiko-chemische Verhalten des in Hefen sezernierten AT III dem aus Humanplasma gewonnenen AT III.

Das so gewonnene AT III zeigte im Immundiffusionstest (Ouchterlony, Progr. Allergy 5 (1958) 1) immunologische Identität mit einem aus Humanserum gewonnenen AT III. Die Progressivinhibitoraktivität (Schrader et al., Ärztl. Lab. 29 (1983) 35 - 39) konnte in vollem Ausmaß nachgewiesen werden.

In den folgenden Beispielen beziehen sich Prozentangaben auf das Gewicht, sofern keine andere Definition genannt ist.

Die Figuren dienen zur Erläuterung der Beispiele:

Figur 1 zeigt die Konstruktion des Vektors pMB181 aus dem Plasmid pUC18 und dem Hybridplasmid pßAT6, das die AT III-cDNA enthält.

Figur 2 zeigt die analoge Konstruktion des Vektors pMB179.

Figur 3 zeigt die Konstruktion des Vektors pMB186 aus dem bekannten Plasmid pAAH5 und einem Fragment aus dem Vektor pMB181 (Figur 1).

Figur 4 zeigt die Konstruktion des Vektors pMB187 aus dem bekannten Plasmid pEX-2 und einem Fragment aus dem Vektor pMB179 (Figur 2).

Figur 5 zeigt die Konstruktion des Vektors pMB218 aus dem Plasmid pFL20 und dem Vektor pMB186 (Figur 3).

Figur 6 zeigt die analoge Konstruktion des Vektors pMB220 mit Hilfe des Vektors pMB187 (Figur 4).

Die Figuren sind nicht maßstabsgerecht, insbesondere im Bereich der Polylinker ist der Maßstab "gedehnt".

In den Beispielen wurden die folgenden Plasmide eingesetzt:

a) handelsübliche Plasmide pUC13 und pUC18 (z.B. "Molecular Biologicals", Chemicals and Equipment for Molecular Biology, Pharmacia, Molecular Biology Division, 1984, 107M84 MBC, Seiten 58 - 59)

b) literaturbekannte Plasmide

YEp13 (US-PS 4 546 082; Broach et al., a.a.O.)

pAAH5 (Ammerer, a.a.O.)

pEX-2 (Ernst und Chan, a.a.O.)

pFL20 (Losson und Lacroute, Cell 32 (1983) 371 - 377)

Die Wirtsstämme sind handelsüblich bzw. bei den anerkannten Hinterlegungsstellen erhältlich.

## Beispiel 1

### Isolierung und Charakterisierung der AT III-cDNA

#### a) Isolierung der m-RNA aus Leber

Gefrorenes humanes Leberbiopsiematerial wurde in flüssigem Stickstoff im Mörser pulverisiert. Das Pulver wurde mit 70°C warmer 4 M Guanidiniumthiocyanat-Stammlösung (J. Chirgwin et al., Biochemistry 18, (1979), 5294 - 5299) in einem Mixer eine Minute lang homogenisiert. Das Homogenat wurde 10 Minuten bei 10°C zentrifugiert (Sorvall-Rotor HB-4, 8000 Upm). Pro ml Überstand wurden 0,5 g Caesiumchlorid zugesetzt und die Mischung einige Minuten auf 60°C erwärmt. Die Lösung wurde auf ein Kissen, bestehend aus 5,7 M Caesiumchlorid, 12,5 mM EDTA (pH 7,5) und 12,5 mM Natriumcitrat (pH 7,0) geschichtet und 17 Stunden bei 20°C zentrifugiert (Beckman SW-28 Rotor, 21000 Upm). Das Pellet wurde in TES-Puffer (50 mM Tris-HCl, 10 mM EDTA, 0,2 % SDS (Natriumdodecylsulfat) pH 7,4) unter Erwärmen suspendiert und einmal mit dem gleichen Volumen einer Mischung aus Chloroform und n-Butanol (4:1, v/v) extrahiert. Nach Zentrifugation wurde die wäßrige Phase mit 1/10 Volumen 3 M Natriumacetat-Lösung (pH 5,2) und dann mit 2 Volumen Ethanol versetzt. Nach mehrstündigem Stehen bei -20°C wurde zentrifugiert und der Niederschlag mit 80 %igem Ethanol gewaschen. Das getrocknete Präzipitat wurde in 0,1 M Natriumacetatlösung (pH 7,0) gelöst und erneut mit Ethanol gefällt. Nach Zentrifugation, Waschen und Trocknen (wie oben) wurde das Präzipitat in 0,1 M Natriumacetat (pH 5,2), 10 mM Tris-HCl (pH 7,4), 1 mM EDTA, 0,2 % (w/v) SDS gelöst und zweimal mit dem gleichen Volumen Phenol (äquilibriert mit 1 M Tris-HCl (pH 8,0) und 0,2 % (w/v) Hydroxychinolin) extrahiert und zentrifugiert. Nach einmaliger Extraktion mit dem gleichen Volumen Chloroform und Zentrifugieren wurde die wäßrige Phase mit dem gleichen Volumen einer Lösung aus Harnstoff (8 M) und Lithiumchlorid (4 M) versetzt und über Nacht bei 0°C aufbewahrt. Nach Zentrifugation (0°C, 10000 Upm, Rotor SS 34) wurde der Niederschlag in TES-Puffer aufgenommen, mit 1/10 Volumen 3 M Natriumacetat-Lösung (pH 5,6) versetzt und mit Ethanol gefällt. Der Niederschlag wurde schließlich in Pufferlösung (50 mM Tris-HCl, 1 mM EDTA, 0,1 % SDS, pH 7,5) aufgenommen, mit dem gleichen Volumen an 1 M NaCl versetzt und die Poly(A)-RNA durch zweimalige Chromatographie an Oligo(dT)-Cellulose (H. Aviv und P. Leder, Proc. Natl. Acad. Sci. USA 69 (1972) 1408 - 1412) isoliert. Die Poly(A)-RNA wurde nach Zugabe von Natriumacetatlösung mit Ethanol gefällt und schließlich in einer Konzentration von 1 µg/µl in 1 mM Tris-HCl, 0,1 mM EDTA (pH 7,0) gelöst.

b) Synthese, Klonierung und Screening von Leber-cDNA

10 µg Poly(A)-RNA in 10 µl des zuletzt genannten Puffers wurden 3 Minuten auf 70°C erwärmt, in Eiswasser abgeschreckt und zur Synthese der cDNA eingesetzt. Die Synthese des ersten Stranges in 100 µl Endvolumen erfolgte nach der Methode von Wickens et al. (J. Biol. Chem. 253 (1978) 2483 - 2495) unter Verwendung von $^{32}P$-markiertem Thymidintriphosphat, wobei zusätzlich der RNase-Inhibitor RNasin (100 U pro Ansatz, Firma Biotec) eingesetzt wurde. Bei der Synthese des zweiten Stranges (ebenfalls nach Wickens et al., a.a.O.) wurde das Klenow-Fragment der DNA-Polymerase I von E. coli (100 U) in einem 200 µl Reaktionsansatz verwendet und die Reaktion 4 Stunden bei 15°C und anschließend 2 Stunden bei 20°C durchgeführt. Durch Extraktion mit einem Volumen Phenol-Chloroform (1:1) wurde die Reaktion gestoppt. Die wäßrige Phase wurde mit 1/10 Volumen 3M Natriumacetat (pH 5,6) versetzt und anschließend wurde mit Ethanol gefällt. Zur Entfernung der "Hairpin"-Strukturen wurde die cDNA 30 Minuten bei 37°C mit 90 Einheiten Nuclease $S_1$ (P-L Biochemi cals) in 250 µl Reaktionsvolumen behandelt (Maniatis et al. (1982) "Molecular Cloning", A Laboratory Manual; Cold Spring Harbor). Die Reaktion wurde durch Extraktion mit Phenol-Chloroform (1:1) gestoppt und die DNA nach Zugabe von Natriumacetat-Lösung mit Ethanol gefällt. Die Enden der cDNA wurden in bekannter Weise mit Hilfe von Klenow-Polymerase (Maniatis et al., a.a.O.) repariert, die Reaktion durch Extraktion mit Phenol-Chloroform (1:1) gestoppt und die DNA, wie oben beschrieben, gefällt.

Zur Transformation von E. Coli HB 101 wurde das Plasmid pUC13 nach Standardmethoden mit der Restriktionsendonuclease SmaI gespalten und mit alkalischer Phosphatase aus Kälberdarm behandelt. Die gewonnene cDNA wurde ohne weitere Vorbehandlung in 1 mM Tris-HCl (pH 7,5) 0,1 mM EDTA gelöst und in den Vektor pUC13 ligiert. (Maniatis et al. a.a.O.)

Die Transformation in E. coli HB 101 erfolgte nach beschriebenen Methoden (Maniatis et. al., a.a.O.). Die Ampicillin-resistenten Kolonien wurden auf Agarplatten mit Nitrocellulosefiltern angezogen. Replica-Platten wurden mit Chloramphenicol (150 µg/ml) behandelt. Die Lyse der Kolonien und die Fixierung der DNA auf den Nitrocellulosefiltern wurden nach publizierten Methoden durchgeführt (Maniatis et al., a.a.O.).

Zur in situ-Hybridisierung wurden die Nitrocellulosefilter 2 Stunden bei 42°C in "prewashing solution" (Maniatis et al., S. 326) inkubiert und anschließend mehrere Stunden bei 42°C in folgendem Puffer vorhybridisiert: 0,9 M NaCl, 0,18 M Tris-HCl (pH 8,0), 6 mM EDTA, 5 x Denhardt-Lösung, 0,2 % SDS, 200 µg/ml gescherte und denaturierte Kalbsthymus-DNA, 200 µg/ml Hefe-RNA, 0,5 % nichtionisches Tensid (Nonidet P-40, Sigma).

Die Hybridisierung erfolgte mit folgender $^{32}P$-markierter DNA-Sequenz im zuletzt erwähnten Puffer ($10^5$ cpm/ml; 16 Stunden bei 42°C):
3'-(AGAAGTACCCGTCTCATCGGTTGGG)-d 5'.

Das Oligonukleotid wurde mittels der Phosphotriestermethode synthetisiert. Die DNA-Sequenz ist komplementär zu den Nukleotiden 1359 - 1383 der AT III-cDNA (Chandra et al., Proc. Natl. Acad. Sci. USA 80 (1983) 1845 - 1848). Die Filter wurden anschließend wie folgt gewaschen: 2 x 20 min bei Raumtemperatur, dann 2 x 20 min bei 33°C in 0,9 M NaCl, 0,09 M Natriumcitrat (pH 7,0). Die Filter wurden getrocknet und zur Detektion von positiven Klonen bei -70°C auf Röntgenfilmen exponiert.

Mehrere hybridisierungspositive Kolonien konnten isoliert werden. Die Plasmid-DNA dieser Kolonien wurde nach Standardverfahren isoliert und durch Spalten mit verschiedenen Restriktionsenzymen charakterisiert. Mehrere der Plasmide enthielten einklonierte Fragmente, die ihrem Restriktionsmuster nach AT III-cDNA enthielten. Einige der AT III-cDNA enthaltenden Plasmide wurden durch Sequenzieren nach Maxam und Gilbert (Methods in Enzymology 65 (1980), 499 - 560) charakterisiert. Die Aminosäuresequenz, für die die AT III-cDNA im Plasmid pßAT6 kodiert, entsprach der'inder EP-A 90 505 bzw. der U S -A 4 517 294 veröffentlichten Sequenz.

Beispiel 2

Subklonierung von AT III-cDNA

Das ca. 1400 bp große EcoRI-HindIII-Fragment (2) aus pßAT6 (1), das die kodierende Sequenz für AT III enthält, wurde isoliert, die überstehenden 5'-Enden der DNA mit Hilfe der Polymerase I (Klenow-Fragment) zur DNA-Sequenz (3) aufgefüllt und der HindIII-8mer-Linker (4)
5'd-(CAAGCTTG)-3 (4)

an das DNA-Ende ligiert, wobei die DNA-Sequenz (5) erhalten wird. Anschließend wurde die DNA (5) mit HindIII verdaut und das ca. 1400 bp große DNA-Fragment (6) in pUC18, das mit HindIII linearisiert wurde (7), ligiert. Das neue Plasmid pMB181 (8) enthält die AT III-cDNA, flankiert von HindIII-Schnittstellen (Fig. 1).

Um die AT III-cDNA als "portfables" BamHI-Fragment zu erhalten, wurde an das ca. 1400 bp große, aufgefüllte EcoRI-HindIII-Fragment (3) aus pßAT6 der BamHI-12mer-Linker (9)

5'-d-(CGCGGATCCGCG)-3' (9)

unter Bildung der DNA-Sequenz (10) anligiert, nach Schneiden mit BamHI das Fragment (11) isoliert und in pUC18 kloniert, das mit BamHI zu (12) linearisiert wurde. In dem neuen Plasmid pMB179 (13) liegt die AT III-cDNA flankiert von BamHI-Schnittstellen vor (Fig. 2).

Die Plasmide pMB181 und pMB179 wurden in dem E. coli-Stamm D29A1 (Δlac-pro, $Nal^R$, $thia^-$, SuII, $hsdr^+$, $hsdM^+$ (F'$lacI^q$ ZM 15 $pro^+$)) transformiert und dort amplifiziert.

## Beispiel 3

Konstruktion von AT III-cDNA-Expressionsvektoren für S. cerevisiae

Als Expressionsplasmide dienten E. coli-S. cerevisiae-"shuttle"-Vektoren, die Sequenzen von pMB9 oder pBR322 enthalten. Sie werden somit in E. coli repliziert und besitzen als Selektionsmarker für E. coli das ß-Lactamasegen. Als Selektionsmarker für Hefen dienten entweder das Leu2-Gen, das für das Enzym 3-Isopropylmalatdehydrogenase kodiert, oder das Ura-3-Gen, das die genetische Information für das Enzym Orotidin-5'-phosphatdecarboxylase beeinhaltet. Teile der S. cerevisiae-2μ-DNA auf den Expressionsvektoren gewährleisten die Replikation der Plasmide und eine hohe Kopienzahl in Hefen. Die Medien und Kulturbedingungen für das Wachstum von Hefen sind beschrieben in Dillon et al. ((1985), Recombinant DNA Methodology, John Wiley & Sons, New York).

### a) Vektoren mit Alkoholdehydrogenase I-Promotor (ADH I)

Die AT III-cDNA wurde als HindIII-Fragment (6) (Fig. 1) in die HindIII-Stelle des Vektors pAAH5 (14) kloniert (Fig. 3). In dem neuen Plasmid pMB186 (15) steht die AT III-cDNA somit unter der Kontrolle des starken ADH I-Promotors, der die Genexpressionssignale der Alkoholdehydrogenase I enthält. Die Effizienz des ADH I-Promotors ergibt sich daraus, daß die ADH I-mRNA ca. 1 - 2 % der gesamten Poly(A)-RNA in S. cerevisiae darstellt. S. cerevisiae, Stamm Pep4-3, $Leu2\text{-}3^-$, wurde nach der Methode von Itoh et al. (J. Bacteriol. 153 (1983) 163 - 168) in Gegenwart von LiCl mit (15) transformiert und $Leu^+$-Transformanten auf YNB-Minimalmedium selektioniert.

### b) Vektoren mit Cytochrom I-Promotor (cyc I)

Das 1400 bp große BamHI-Fragment aus (11) (Fig. 2) wurde in pEX-2 (16), das mit BamHI linearisiert worden war, ligiert. In dem neuen Plasmid pMB187 (17) (Fig. 4) steht die AT III-cDNA unter der Kontrolle des cycI-Promotors. S. cerevisiae, Stamm YNN27 (Ura3-52, trpl-289, gal2), wurde mit (17) wie unter a) transformiert und $Ura^+$-Transformanten auf YNB-Medium, dem Tryptophan in der Konzentration von 30 μg/ml zugesetzt worden war, selektioniert.

## Beispiel 4

Integration der AT III-cDNA-Sequenz in chromosomale DNA von Hefen

Das ca. 3350 bp große BamHI-Fragment (18) aus pMB186 (15), das die für AT III codierende cDNA enthält, wurde in die Hpa I-Stelle des Plasmides YEp13 benachbart der Sequenz für das Leu2-Strukturgen ligiert, nachdem die Strangenden jeweils mit Klenow-Fragment der DNA Polymerase I aufgefüllt waren. Anschließend wurde das ca. 5700 bp große XhoI-SalI-Fragment, das die Information für das Leu2-Gen und die AT III-cDNA trägt, isoliert und S. cerevisiae (Pep 4-3, Leu 2-3) mit diesem DNA-Fragment transformiert. Es wurden sieben Klone isoliert, die durch Rekombinationsereignisse den $Leu^+$-Phänotyp aufwiesen. Alle Klone erwiesen sich, da sie das Leu2-Gen und die AT III-cDNA chromosomal integriert haben, als stabil hinsichtlich der Leu-Prototrophie und der AT III-Synthese und können ohne Verlust dieser Eigenschaften in nicht selektiven Medien fermentiert werden.

Beispiel 5

Konstruktion von AT III-Expressionsvektoren für Schizosaccharomyces pombe

Die Spalthefe S. pombe ist eine genetisch gut untersuchte Hefe, für die in der letzten Zeit Vektoren entwickelt wurden, die sich als Episom stabil in der Zelle replizieren. Zu diesen Plasmiden zählt das pFL20, das neben einer autonomen Replikationssequenz, die die Replikation in S. pombe ermöglicht, eine DNA-Sequenz enthält, die die Stabilität des Plasmids während der Mitose und Meiose gewährleistet.

a) S. pombe-Expressionsvektoren mit dem S. cerevisiae-ADH I Promotor

In pFL20 (19), das mit BamHI linearisiert wurde, wurde das ca. 3350 bp große BamHI-Fragment (18) aus pMB186 kloniert (Fig. 5). Somit enthält das neue Plasmid pMB218 (20) alle notwendigen Elemente zur stabilen Replikation in S. pombe sowie die AT III-cDNA unter Kontrolle des S. cerevisiae ADH I-Promotors. S. pombe, Stamm 972 $h^-$ $Ura^-$, wurde mit (20) mit Lithiumacetat nach der Methode von Itoh et al. (a.a.O.) transformiert und $Ura^+$-Transformanten auf YNB-Agar selektioniert.

b) S. pombe-Expressionsvektoren mit dem S. cerevisiae-cycI-Promotor

Das ca. 2500 bp SalI-Fragment (21) aus pMB187 (17) (Fig. 4), das den S. cerevisiae-cycI-Promotor und die AT III-cDNA enthält, wurde nach Auffüllen der überstehenden Enden zu (23) unter "blunt end"-Bedingungen in pFL20 (19), das mit NruI und PvuII zu (22) linearisiert wurde, einligiert (Fig. 6). Somit steht in dem neuen Plasmid pMB220 (24) die AT III-cDNA unter der Kontrolle des S. cerevisiae-cycI-Promotors; das Plasmid ist aber in S. pombe stabil replizierbar. S. pombe wurde mit pMB220 ebenso transformiert wie mit pMB218 (20) und Klone auf YNB-Agar selektioniert.

Beispiel 6

Synthese von AT III in Hefen

a) Synthese von AT III in S. cerevisiae

S. cerevisiae (Pep4-3 Leu 2-3, pMB186) und S. cerevisiae (YNN27; pMB187) wurden in selektiven Minimalmedium angezogen und anschließend auf YPD-Medium überimpft. Im Kulturfiltrat beider Kulturen wurde AT III mittels eines ELISA, der spezifisch auf humanes AT III gerichtet ist, in Konzentrationen von 20 - 40 ng/ml nachgewiesen, während intrazellulär nur 2 - 10 ng/ml Zellsuspension nachgewiesen werden konnten (siehe auch Tabelle 1). Somit wird der größte Teil des AT III prozessiert und ausgeschieden.

Die Festphase des auf humanes ATIII gerichteten ELISA bestand aus Polystyrolröhrchen beschichtet mit anti-ATIII Immunoglobulin, das durch Affinitätschromatographie aus Seren von mit 99 % reinem humanem ATIII immunisierten Kaninchen isoliert wurde. Verwendung als Konjugat fanden Anti-Kaninchen-Immunoglobulin-Antikörper aus der Ziege, die mit Meerrettich-Peroxidase konjugiert waren. Im Bereich von 2-100 ng/ml waren die Eichkurven linear. Bei Testung von Hefezellextrakten oder Hefekulturüberständen wurde keine unspezifische Reaktion beobachtet.

b) Synthese von AT III in S. pombe

In S. pombe ($Ura^-$; pMB218) und S. pombe ($Ura^-$, pMB 220) konnte mittels des AT III-spezifischen ELISA die Synthese von AT III nachgewiesen werden. Bei Fermentation im YPD-Medium sezernierten die Zellen in batch-Kultur bis zu 70 ng AT III/ml. Somit wird die humane AT III-Signalsequenz auch von S. pombe erkannt.

Beispiel 7

Reinigung und Charakterisierung des von Hefen produzierten humanen AT III

Humanes AT III wurde aus Überständen von Hefekulturen gemäß Beispiel 3 bis 6 mittels Affinitätschromatographie an Heparin-SEPHAROSE angereichert (Miller-Anderssen et al., a.a.O.). Das aus Hefen isolierte AT III zeigte im Immundiffusionstest nach Ouchterlony (a.a.O.) immunologische Identität mit dem aus Humanserum gewonnenen AT III. Für das von Hefen produzierte AT III konnte Progressivinhibitoraktivität (Schrader et al., a.a.O.) in vollem Ausmaß nachgewiesen werden. Ebenso wurde Heparin Cofaktor-Aktivität nachgewiesen.

Vergleichsbeispiel

Analog zum Plasmid pMB186 wird ein Plasmid konstruiert, das anstelle der AT III-Präsequenz für die Sequenz
Met-Ala-Cys-Ser-Ser-Arg-Val-Asp-Asp-Pro
ATG GCT PGC TCC PCT AGA GTC GAC GAC CCT
kodiert (pMB 205).

Transformiert man S. cerevisiae (Pep4-3) mit pMB205, so bilden die Zellen ein modifiziertes AT III ohne Signalsequenz. Dieses kann nur im Zellextrakt, nicht aber im Überstand nachgewiesen werden.

Tabelle 1

Synthese von AT III in Hefen

| Hefe | Plasmid | Promotor | AT III im Medium (ng/ml) | AT III intrazellulär (ng/ml) |
|---|---|---|---|---|
| S. cerevisiae | pMB186 | ADH I | 20 | 5-10 |
| S. cerevisiae | pMB187 | cyc I | 30 | 7-10 |
| S. pombe | pMB218 | ADH I | 70 | 10 |
| S. pombe | pMB220 | cyc I | 50 | 14 |
| S. cerevisiae | chromosomal integriert | ADH I | 10-15 | 5 |
| S. cerevisiae | pMB205 | ADH I | 0 | 10 |

**Ansprüche**

1. Verfahren zur Sekretion von Proteinen aus Hefen, dadurch gekennzeichnet, daß man die Signalsequenz für humanes Antithrombin III (AT III) oder Teile derselben verwendet.

2. Verfahren nach Anspruch 1, dadurch gekennzeichnet, daß ein Fusionsprotein mit AT III oder Teilen von AT III sekretiert wird.

3. Verfahren nach Anspruch 1, dadurch gekennzeichnet, daß als Strukturgen die cDNA-Sequenz für AT III eingesetzt wird.

4. Verfahren nach Anspruch 1, 2 oder 3, dadurch gekennzeichnet, daß eine Hefe der Familie Saccharomycetaceae eingesetzt wird.

5. Verfahren nach Anspruch 1, 2, 3 oder 4, dadurch gekennzeichnet, daß eine Hefe der Art Saccharomyces cerevisiae oder Schizosaccharomyces pombe eingesetzt wird.

6. Biologisch aktives AT III, erhältich nach einem oder mehreren der vorhergehenden Ansprüche.

7. Arzneimittel, enthaltend AT III gemäß Anspruch 6.

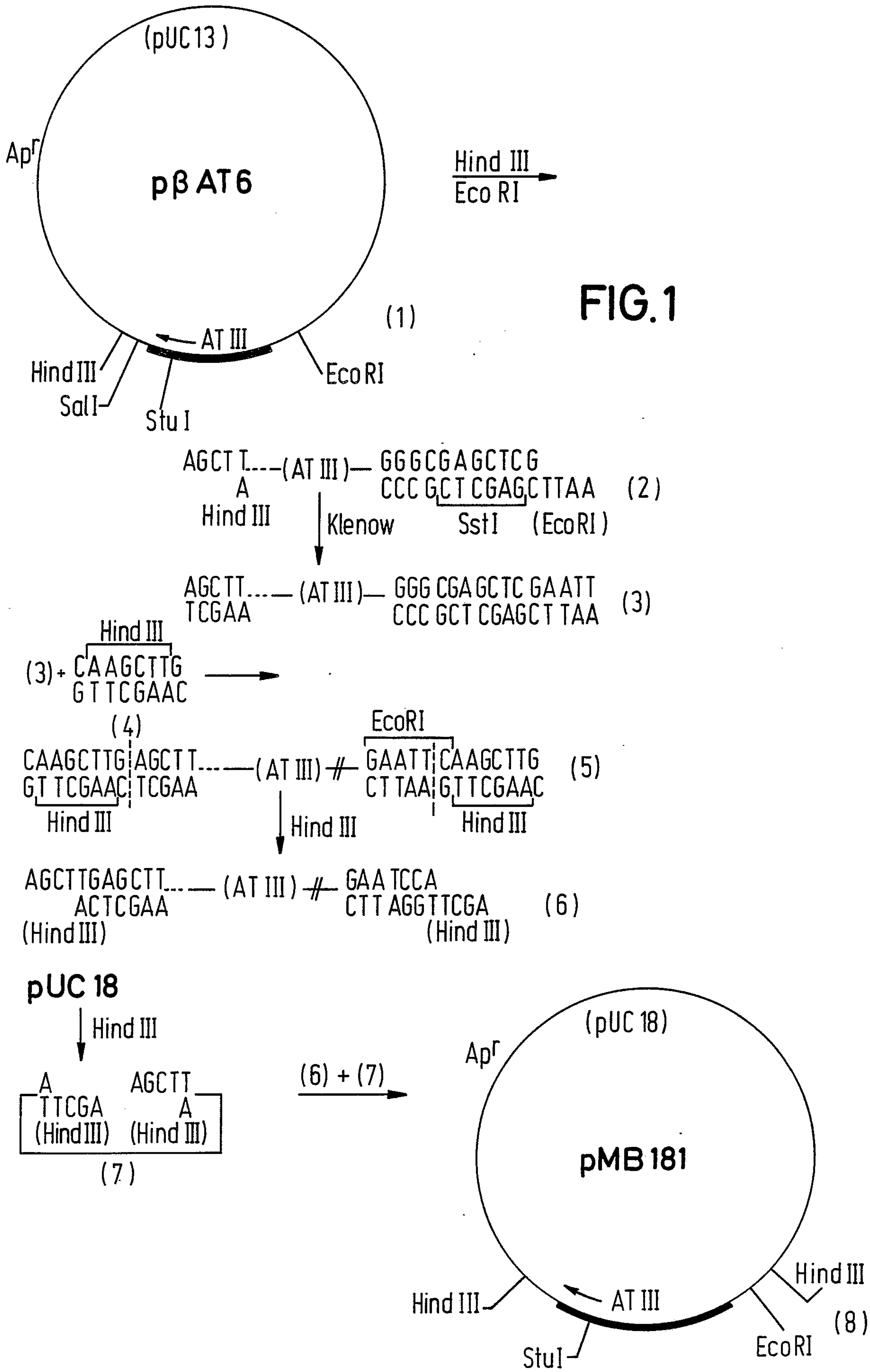
(pUC13)
Apr
pβ AT6
Hind III
Eco RI
(1)
AT III
Hind III
Sal I
Stu I
Eco RI
AGCTT----(AT III)— GGGCGAGCTCG
A
CCCGCTCGAGCTTAA
(2)
Hind III
Klenow
Sst I
(EcoRI)
AGCTT----(AT III)— GGGCGAGCTCGAATT
TCGAA
CCCGCTCGAGCTTAA
(3)
Hind III
(3) + CAAGCTTG
GTTCGAAC
(4)
EcoRI
CAAGCTTG AGCTT----(AT III) GAATT CAAGCTTG
GTTCGAAC TCGAA CTTAA GTTCGAAC
(5)
Hind III
Hind III
Hind III
AGCTTGAGCTT----(AT III) GAATCCA
ACTCGAA
CTTAGGTTCGA
(6)
(Hind III)
(Hind III)
pUC 18
Hind III
A
TTCGA
(Hind III)
AGCTT
A
(Hind III)
(7)
(6) + (7)
(pUC 18)
Apr
pMB 181
Hind III
Hind III
AT III
Stu I
Eco RI
(8)

FIG.1

FIG. 2

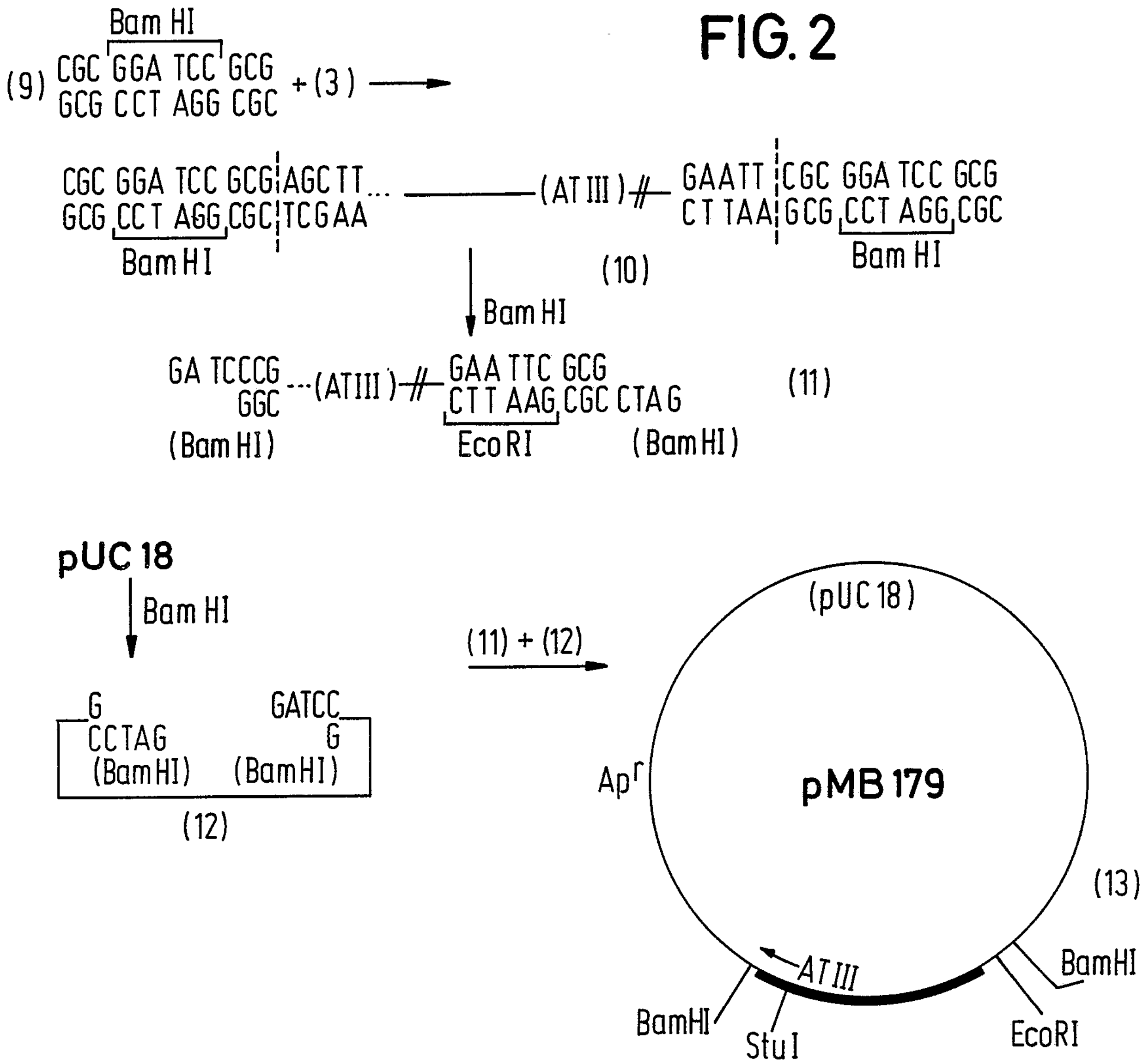

Bam HI
(9) CGC GGA TCC GCG
GCG CCT AGG CGC
+ (3)
CGC GGA TCC GCG AGC TT...
GCG CCT AGG CGC TCGAA
Bam HI
(AT III)
GAATT CGC GGA TCC GCG
CT TAA GCG CCT AGG CGC
Bam HI
(10)
Bam HI
GA TCCCG
GGC
(AT III)
GAA TTC GCG
CTT AAG CGC CTAG
(11)
(Bam HI)
Eco RI
(Bam HI)
pUC 18
Bam HI
G
CCTAG
(Bam HI)
GATCC
G
(Bam HI)
(12)
(11) + (12)
(pUC 18)
Ap^r
pMB 179
(13)
AT III
BamHI
BamHI
Stu I
EcoRI


FIG. 3

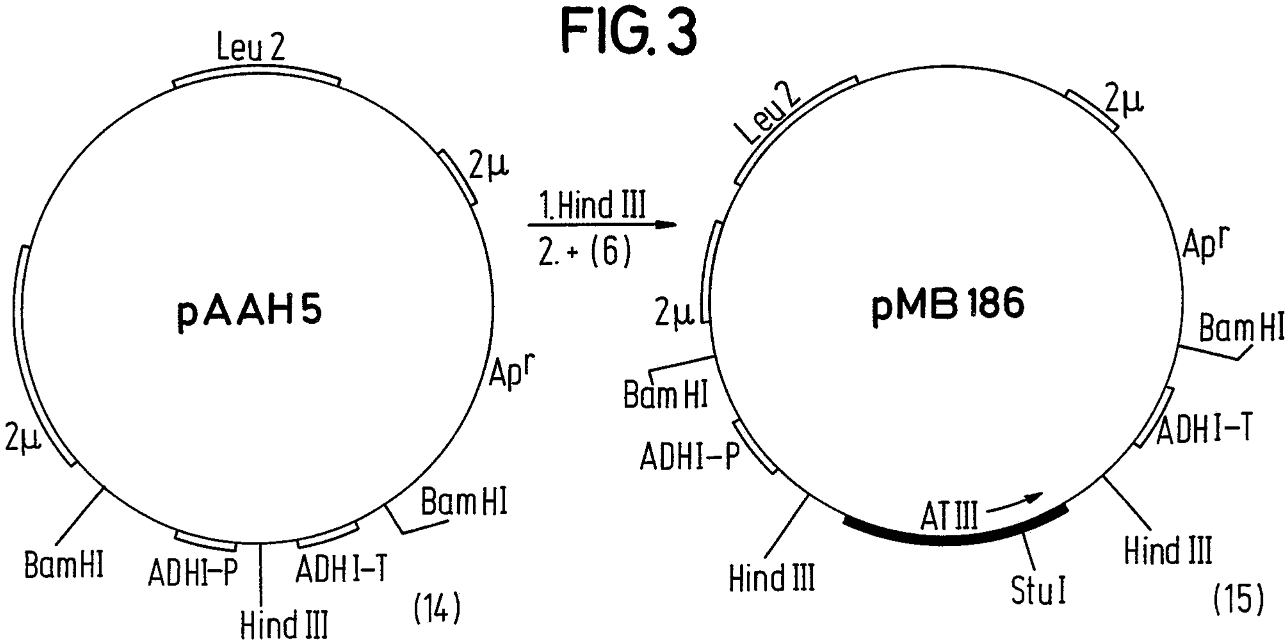

Leu 2
2µ
2µ
pAAH5
Ap^r
BamHI
BamHI
ADHI-P
ADHI-T
Hind III
(14)
1. Hind III
2. + (6)
Leu 2
2µ
2µ
pMB 186
Ap^r
BamHI
Bam HI
ADHI-P
ADHI-T
AT III
Hind III
Hind III
Stu I
(15)

FIG.4

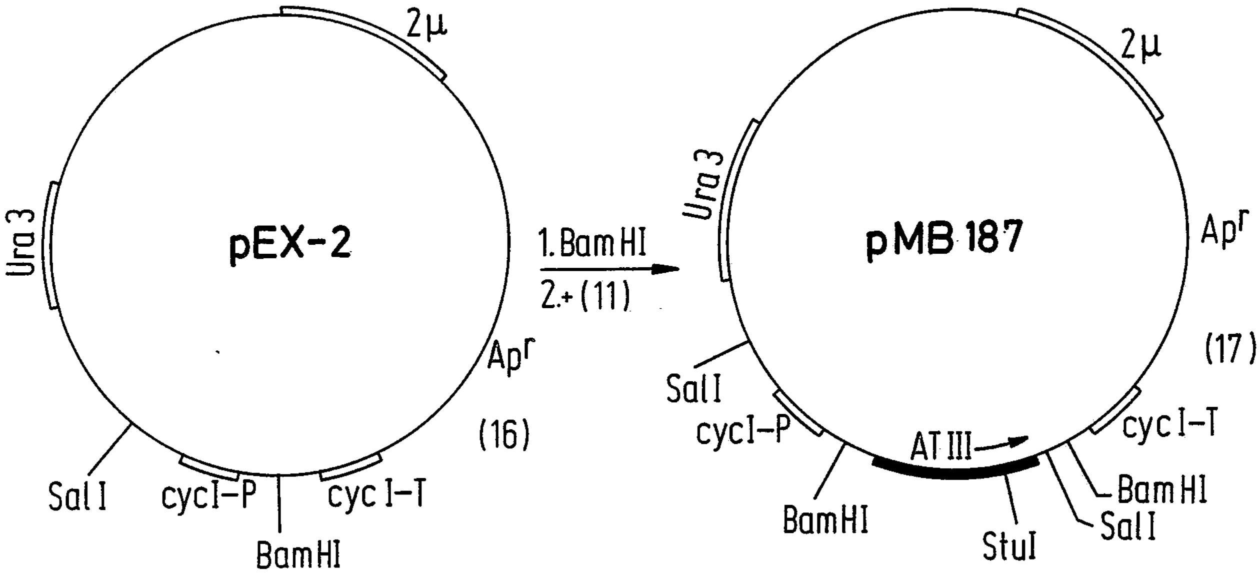
2μ
Ura 3
pEX-2
Apr
Sal I
cycI-P
BamHI
cyc I-T
(16)
1. BamHI
2.+ (11)
2μ
Ura 3
pMB 187
Apr
(17)
Sal I
cycI-P
BamHI
AT III
StuI
cyc I-T
BamHI
Sal I

FIG.5

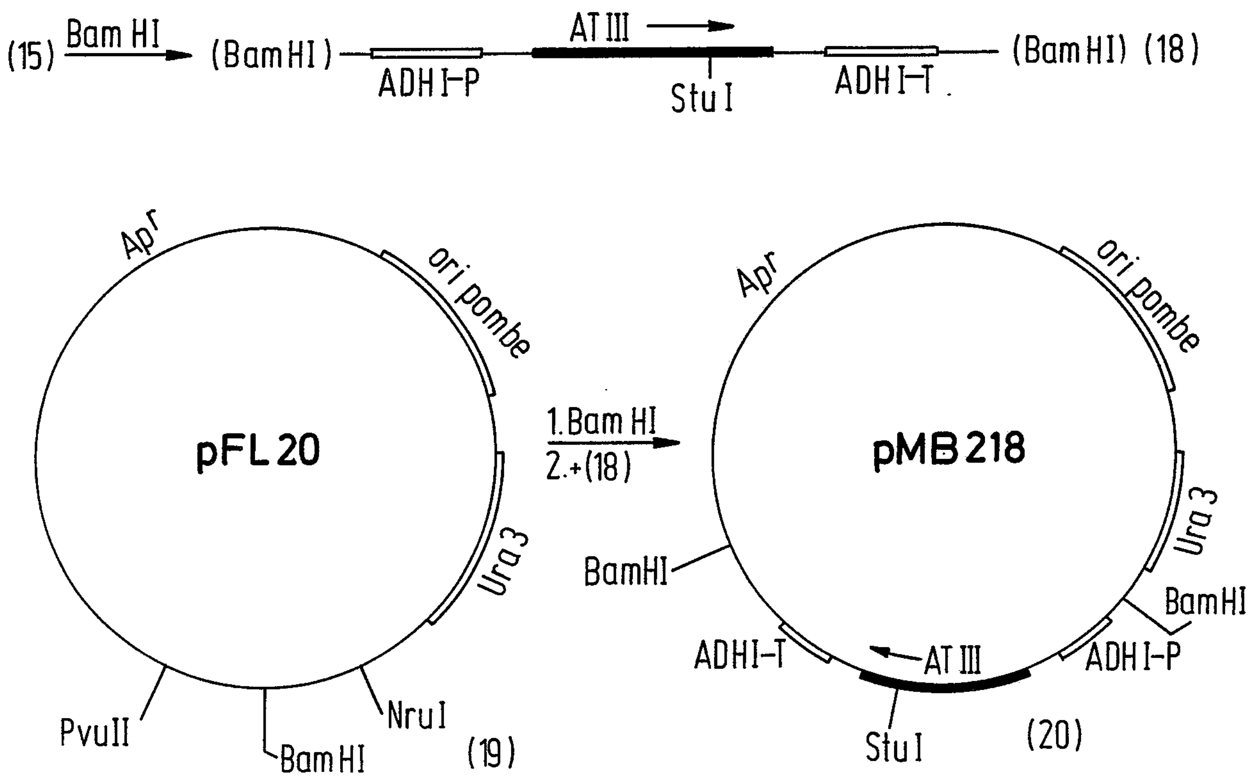
(15)
Bam HI
(BamHI)
ADH I-P
AT III
Stu I
ADH I-T
(BamHI) (18)
Apr
ori pombe
pFL 20
Ura 3
PvuII
BamHI
NruI
(19)
1. Bam HI
2.+(18)
Apr
ori pombe
pMB 218
Ura 3
BamHI
ADHI-T
AT III
StuI
ADHI-P
BamHI
(20)

FIG.6

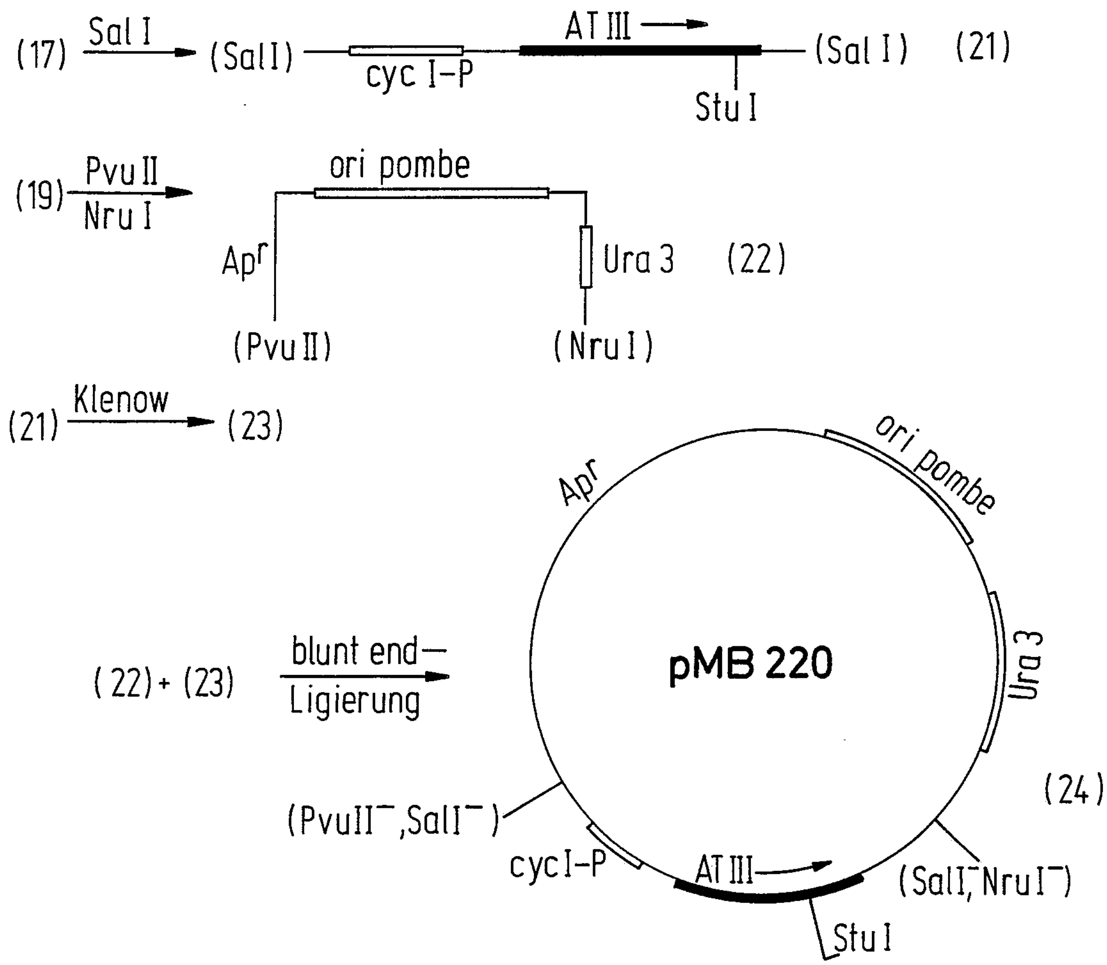

(17)
Sal I
(SalI)
cyc I-P
AT III
Stu I
(Sal I)
(21)
(19)
Pvu II
Nru I
ori pombe
Apr
Ura 3
(22)
(Pvu II)
(Nru I)
(21)
Klenow
(23)
(22) + (23)
blunt end-
Ligierung
ori pombe
Apr
pMB 220
Ura 3
(24)
(PvuII⁻, SalI⁻)
cycI-P
AT III
(SalI⁻, NruI⁻)
Stu I

Europäisches Patentamt

# EUROPÄISCHER RECHERCHENBERICHT

Nummer der Anmeldung

EP 87107795.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | EP - A2 - 0 090 505 (GENENTECH, INC.)<br>* Zusammenfassung; Ansprüche 1,12,14 * | 6,7 | C 12 N 15/00<br>C 07 K 15/14<br>C 12 P 21/00<br>A 61 K 37/02<br>//(C 12 N 15/00;<br>C 12 R 1:85)<br>(C 12 N 15/00;<br>C 12 R 1:865) |
| A | * Zusammenfassung *<br>---- | 1 | |
| | | | **RECHERCHIERTE SACHGEBIETE (Int. Cl 4)** |
| | | | C 12 N<br>C 07 K<br>C 12 P<br>A 61 K |

Der vorliegende Recherchenbericht wurde für alle Patentansprüche erstellt.

| Recherchenort | Abschlußdatum der Recherche | Prüfer |
|---|---|---|
| WIEN | 01-09-1987 | WOLF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82